Europäisches Patentamt

European Patent Office

Office européen des brevets

(11) Veröffentlichungsnummer: **0 332 887**
**A2**

## (12) EUROPÄISCHE PATENTANMELDUNG

(21) Anmeldenummer: **89103001.7**

(22) Anmeldetag: **21.02.89**

(51) Int. Cl.4: **A61C 13/08 , A61K 6/00**

(30) Priorität: **17.03.88 DE 3809019**

(43) Veröffentlichungstag der Anmeldung:
**20.09.89 Patentblatt 89/38**

(84) Benannte Vertragsstaaten:
**AT CH DE ES FR GB IT LI SE**

(71) Anmelder: **Müterthies, Klaus**
**Adenauer Strasse 69**
**D-4834 Harsewinkel(DE)**

(72) Erfinder: **Müterthies, Klaus**
**Adenauer Strasse 69**
**D-4834 Harsewinkel(DE)**

(74) Vertreter: **Wächtershäuser, Günter, Dr.**
**Tal 29**
**D-8000 München 2(DE)**

(54) Verfahren zur Herstellung von metallkeramischen Dentalrestaurationen sowie Keramik-Farbsystem zur Durchführung des Verfahrens.

(57) Es wird ein Verfahren zur Herstellung von metallkeramischen Dentalrestaurationen beschrieben. Zur Erzielung einer optimalen ästhetischen Anpassung der Dentalrestauration an das Restgebiß werden individuelle Charakteristika des natürlichen, zu ersetzenden Zahns bzw. der natürlichen benachbarten Zähne im Zuge des Aufbaus der metallkeramischen Dentalrestauration durch gezieltes Einlegen oder Zumischen einer keramischen Malfarbe zu der Dentinmasse und/oder der Schmelzmasse nachgebildet. Bei Anwendung des erfindungsgemäßen Verfahrens kann die Anzahl der erforderlichen, unterschiedlich pigmentierten und getrübten Keramikmassen drastisch reduziert werden.

EP 0 332 887 A2

## Verfahren zur Herstellung von metallkeramischen Dentalrestaurationen sowie Keramik-Farbsystem zur Durchführung des Verfahrens

Die Erfindung betrifft ein Verfahren zur Herstellung von metallkeramischen Dentalrestaurationen gemäß dem Oberbegriff von Anspruch 1.

Es ist bereits bekannt, beschädigte Zähne durch Beschleifen und Überkronen zu restaurieren. Bei extrahierten Zähnen kommen Metallbrücken zum Einsatz. Dabei dienen die benachbarten, beschliffenen Zähne als Brückenpfeiler, und an die Stelle des verlorenen Zahnes tritt ein metallisches Zwischenglied. Um der Restauration das Aussehen des natürlichen Zahnes zu verleihen, können die sichtbaren Metallteile mit einer emaille-ähnlichen Keramikschicht verblendet werden. Ein spezielles Problem ist dabei die ästhetische Anpassung der Dentalrestauration an das Restgebiß.

Die Farbe natürlicher Zähne überdeckt ein weites Spektrum. Bei den bisher bekannten Verfahren hat man versucht, anhand von verschieden gefärbten Musterzähnen, die je nach Fabrikat mit sechzehn bis vierundzwanzig verschiedenen Zahnfarben in einem sogenannten Farbenselektor zusammengefaßt sind, die für den jeweiligen Patienten passendste Farbe auszuwählen.

Bei der Verblendung der sichtbaren Metallteile mit der emaille-ähnlichen Keramikschicht benutzt der Zahntechniker üblicherweise drei verschieden stark getrübte, pulverisierte Keramikmassen. Diese werden nacheinander mit einer Flüssigkeit angeteigt, auf das Metallgerüst aufgetragen und aufgebrannt. Die erste, möglichst stark getrübte Keramikschicht - der Opaquer - soll das gewöhnlich dunkle Metall abdecken. Auf dieser Opaquerschicht werden anschliessend weitere Keramikschichten aufgebracht, die in Trübung und Farbe den natürlichen Zahnsubstanzen - Dentin und Schmelz - nahekommen sollen. Die Zahnfarbe der fertig verblendeten Dentalrestauration resultiert somit bei dem herkömmlichen Verfahren aus dem Zusammenwirken von mindestens drei verschieden pigmentierten und getrübten Keramikpulvern: Opaquer, Dentin und Schmelz. Ein Farbselektor mit zwanzig Zahnfarben erfordert also sechzig verschiedene Keramikpulver. Trotz dieser großen Anzahl verschiedener Keramikpulver gelingt es nicht immer, ein befriedigendes ästhetisches Ergebnis, d. h. einen den individuellen Charakteristika des Patienten optimal angepaßten Zahnersatz, zu erzielen. Es kommt daher häufig zu Reklamationen wegen Farbabweichungen, wodurch eine kostspielige Neuverblendung des Metallgerüstes notwendig wird. Ursachen hierfür sind beispielsweise eine nicht sorgfältig durchgeführte Farbauswahl seitens des Zahnarztes oder Fehler des Zahntechnikers bei der Herstellung der Verblendung.

Bei dem herkömmlichen Verfahren werden jeweils einheitlich gefärbte Opaquer-, Dentin- und Schmelzschichten kombiniert, und man erhält einen einheitlich gefärbten Zahn. Individuelle Charakteristika, wie z. B. Schmelzrisse, Kalkflecken usw. werden gegebenenfalls nach dem Aufbrennen von Opaquer, Dentin und Schmelz aufgemalt, und zwar unter Verwendung von stark pigmentierten Massen, sogenannten Malfarben. Eine derartige Verfahrensweise führt jedoch nicht zu einer optimalen ästhetischen Anpassung der Dentalrestauration an das Restgebiß.

Es ist Aufgabe der vorliegenden Erfindung, ein Verfahren zur Herstellung von metallkeramischen Dentalrestaurationen anzugeben, bei dem man ein besseres ästhetisches Ergebnis, d. h. einen der Natur besser angepaßten Zahnersatz, mit einer erheblich reduzierten Anzahl an Farbmusterzähnen und keramischen Massen herstellen kann.

Diese Aufgabe wird bei einem Verfahren der eingangs genannten Art durch die Merkmale des Anspruchs 1 gelöst.

Bevorzugte Ausführungsformen der Erfindung sind in den Unteransprüchen gekennzeichnet.

Die Ansprüche 5 und 6 betreffen ein bevorzugtes Metallkeramik-Farbsystem zur Durchführung des erfindungsgemäßen Verfahrens.

Es hat sich überraschenderweise gezeigt, daß trotz Verwendung einer wesentlich geringeren Anzahl an verschieden gefärbten Keramikmassen als bei dem herkömmlichen Verfahren eine metallkeramische Dentalrestauration geschaffen werden kann, die in ästhetischer Hinsicht den nach herkömmlichen Verfahren hergestellten Restaurationen überlegen ist, d. h. dem Erscheinungsbild des natürlichen Restgebisses näherkommt.

Das bei dem erfindungsgemäßen Verfahren eingesetzte Metallkeramik-Farbsystem umfaßt vorzugsweise eins bis drei Opaquermassen, zwei bis sechs Dentinmassen, eins bis zwei Schmelzmassen, eine transparente Masse sowie fünf bis zehn stark pigmentierte keramische Malfarben. Mit einem derartigen Farbsystem kann das gesamte Spektrum dem natürlichen Zahnfarben abgedeckt werden, wozu nach dem herkömmlichen Verfahren beispielsweise sechzig verschiedene Massen erforderlich waren. Besonders bevorzugt ist ein Metallkeramik-Farbsystem, das eine Opaquermasse, bis zu vier verschiedene Dentinmassen, eine Schmelzmasse, eine transparente Masse sowie fünf bis zehn stark pigmentierte keramische Malfarben umfaßt.

Der Erfindung liegt die Erkenntnis zugrunde, daß eine der wichtigsten Voraussetzungen für eine unauffällige, natürliche Restauration darin besteht, die Zahnform bzw. die individuellen Charakteristika der zu ersetzenden bzw. zu überkronenden Zähne optimal auf die Zahnform bzw. die individuellen Charakteristika der benachbarten verbliebenen, natürlichen Zähne abzustimmen. Individuelle Charakteristika in diesem Sinne sind z. B. Schmelzrisse, Kalkflecken, bläuliche Schmelzkeile und Schneidekanten. Sobald Oberflächengestaltung und Glanz der Keramik hinsichtlich dieser individuellen Charakteristika der Natur entsprechen, sind kleine Farbabweichungen weniger auffällig und tolerierbar.

Bei dem erfindungsgemäßen Verfahren wird folgendermaßen vorgegangen. Der Zahnarzt entscheidet nur zwischen wenigen, vorzugsweise vier Zahnfarben (Farbmusterzähnen), die annähernd einen gleichen, sonnig-gelben Farbton aufweisen, aber in Helligkeit und Farbsättigung abgestuft sind. Unter diesen wenigen Farbmusterzähnen wird die für den jeweiligen Patienten passendste Zahnfarbe ausgewählt. Zusätzlich macht der Zahnarzt möglichst detaillierte Angabe über Form, Lage und Farbe von individuellen Charakteristika der natürlichen, benachbarten Zähne bzw. der zu ersetzenden Zähne. Diese individuellen Charakteristika wie z. B. Schmelzrisse, Kalkflecken, bläuliche Schmelzkeile und Schneidekanten, werden in eine Schemazeichnung des Zahnes eingetragen. Vorzugsweise erfolgt die Darstellung der individuellen Charakteristika in der Schemazeichnung mit Farbstiften, die den zu verwendenden keramischen Malfarben entsprechen. Es ist von besonderem Vorteil, wenn der ausführende Zahntechniker bei der Herstellung der Schemazeichnung zugegen ist.

Beim Aufbau der metallkeramischen Dentalrestauration nach dem erfindungsgemäßen Verfahren wird zunächst die Opaquerschicht auf übliche Weise aufgebracht. Anschließend werden die individuellen Charakteristika des Zahns entsprechend der Schemazeichnung im Zuge des weiteren Aufbaus der Dentalkeramik nachgebildet. Entscheidend für das Gelingen der Arbeit ist es, daß die charakteristischen Merkmale des individuellen Zahns nicht nach Aufbrennen von Opaquer, Dentin und Schmelz aufgemalt werden, sondern bereits im Zuge des Aufbaus der Metallkeramik vom Beginn an miteinbezogen werden. Dieses Ziel wird dadurch erreicht, daß man eine geeignete keramische Malfarbe, gegebenenfalls vermischt mit einer transparenten Masse, gezielt in die Dentin-und/oder Schmelzmasse einlegt oder zumischt. Dadurch wird erreicht, daß bei der Dentalrestauration die individuellen Zahncharakteristika die gleiche räumliche Wirkung haben, wie sie auch beim natürlichen Zahn vorliegt.

Bei dem erfindungsgemäßen Verfahren können übliche dentalkeramische Massen eingesetzt werden. Typische Zusammensetzungen von geeigneten dentalkeramischen Massen (in Gewichtsprozent) sind in der nachfolgenden Tabelle zusammengestellt.

|  | Opaquermasse | Dentin und Schmelzmasse | Malfarbe |
|---|---|---|---|
| $SiO_2$ | 50 - 65 | 60 - 70 | 60 - 70 |
| $Al_2O_3$ | 10 - 20 | 12 - 18 | 5 - 15 |
| $Li_2O$ | 0 - 1 | 0 - 2 | 0 - 3 |
| $Na_2O$ | 2 - 5 | 2 - 6 | 2 - 8 |
| $K_2O$ | 8 - 12 | 8 - 14 | 2 - 10 |
| $MgO$ | 0 - 2 | 0 - 2 | 0 - 2 |
| $CaO$ | 0 - 5 | 0 - 5 | 0 - 5 |
| $BaO$ | 0 - 2 | 0 - 2 | 0 - 5 |
| $B_2O_2$ | - | 0 - 2 | 0 - 12 |
| Trübungsmittel ($SnO_2$, $CeO_2$ Zr $SiO_4$) | 10 - 20 | 0 - 2 | - |
| Pigmente | 2 - 10 | 0,5 - 3 | 15 - 25 |

Geeignete "Pigmente" sind anorganische, farbige Verbindungen, die sich bei der Aufbrenntemperatur nicht in der Glasschmelze lösen oder farblich verändern. Geeignete "Pigmente" in diesem Sinne sind dem Fachmann bekannt und können anhand von Literaturangaben ohne Schwierigkeiten ausgewählt werden. Es wird beispielsweise verwiesen auf Ullmann's Encyclopädie der Technischen Chemie, Dr. H.J. Krause, Keramische Farben, 4. Auflage, Bd. 14, Seiten 1 bis 12.

Besonders geeignete Pigmente sind beispielsweise Zn(Fe)O • Al $(Cr)_2O_3$ braun; $ZrSi(Pr)O_4$ gelb; Al-$(Cr)_2O_3$ rosa.

Die dentalkeramischen Massen werden üblicherweise in Pulverform, d. h. als Keramikpulver, in den Handel gebracht. Dabei werden die zur Erzielung der gewünschten Zahnfarben erforderlichen, verschieden pigmentierten und getrübten Keramikpulver üblicherweise in einem Sortiment zusammengefaßt. Ein derartiges Sortiment wird als Metallkeramik-Farbsystem bezeichnet. Das neuartige Farbsystem für die Durchführung des erfindungsgemäßen Verfahrens umfaßt vorzugsweise eins bis drei verschieden pigmentierte und getrübte Keramikpulver für die Opaquermassen; zwei bis sechs verschieden pigmentierte und getrübte Keramikpulver für die Dentinmassen; eins bis zwei verschieden pigmentierte und getrübte Keramikpulver für die Schmelzmassen; ein Keramikpulver für die transparente Masse; sowie fünf bis zehn stark pigmentierte Keramikpulver für die Malfarben.

Unter wirtschaftlichen Gesichtspunkten ist es von besonderem Vorteil, daß man das erfindungsgemäße Verfahren mit hervorragendem Erfolg selbst dann durchführt, wenn das Metallkeramik-Farbsystem lediglich ein Keramikpulver für die Opaquermasse, zwei bis vier verschieden pigmentierte und getrübte Keramikpulver für die Dentinmasse, ein Keramikpulver für die Schmelzmasse, ein Keramikpulve für die transparente Masse sowie fünf bis zehn stark pigmentierte Keramikpulver für die Malfarben umfaßt.

Verglichen mit dem Sortiment der Keramikfarben, das für die Durchführung des herkömmlichen Verfahrens erforderlich ist und, wie oben erwähnt, sechzig verschiedene Keramikpulver umfassen kann, ist somit bei dem erfindungsgemäßen Dentalkeramik-Farbsystem die Zahl der erforderlichen Keramikmassen drastisch reduziert. Die mit dieser Reduzierung verbundenen Vorteile liegen auf der Hand.

Die transparente Masse entspricht der Dentin- und der Schmelzmasse in der Zusammensetzung, hat aber keine Pigmente und Trübungsmittel.

Die transparente Masse kann in die Verblendung eingelegt werden, um besonders transparente Partien des natürlichen Zahnes, wie sie etwa im Schneidekantenbereich jugendlicher Zähne auftreten können, zu imitieren.

Außerdem können die Dentin- und Schmelzmassen oder auch die Malfarben mit der transparenten Masse vermischt und dadurch transparenter gemacht werden.

## Ansprüche

1. Verfahren zur Herstellung von metallkeramischen Dentalrestaurationen, wobei man unter Verwendung von mindestens einer Opaquermasse, mindestens einer Dentinmasse, mindestens einer Schmelzmasse sowie gegebenenfalls einer transparenten Masse und stark pigmentierten keramischen Malfarben auf einem Metallsubstrat unterschiedlich pigmentierte und unterschiedlich getrübte Keramikmassen schichtweise aufträgt und nachfolgend brennt, **dadurch gekennzeichnet,** daß man zur Erzielung einer optimalen ästhetischen Anpassung der Dentalrestauration an das Restgebiß individuelle Charakteristika des natürlichen, zu ersetzenden Zahns bzw. der natürlichen, benachbarten Zähne im Zuge des Aufbaus der metallkeramischen Dentalrestauration durch gezieltes Einlegen und/oder Zumischen der keramischen Malfarbe zu der Dentinmasse und/oder der Schmelzmasse darstellt.

2. Verfahren gemäß Anspruch 1, dadurch gekennzeichnet, daß man ein Farbsystem von vorzugsweise bis zu vier Farben verwendet, die annähernd einen gleichen, sonnig-gelben Farbton aufweisen, aber in Helligkeit und Farbsättigung abgestuft sind.

3. Verfahren gemäß einem der Ansprüche 1 oder 2, dadurch gekennzeichnet, daß man die individuellen Charakteristika des Zahns, wie z. B. Schmelzrisse, Kalkflecken, bläuliche Schmelzkeile und Schneidekanten, hinsichtlich Form, Lage und Farbe in eine Schemazeichnung des Zahnes einträgt.

4. Verfahren gemäß einem der Ansprüche 1 bis 3, dadurch gekennzeichnet, daß man die Darstellung der Charakteristika in der Schemazeichnung des Zahnes mit Farbstiften durchführt, die den zu verwendenden keramischen Malfarben entsprechen.

5. Metallkeramik-Farbsystem zur Durchführung des Verfahrens gemäß Anspruch 1, umfassend eins bis drei Opaquermassen, zwei bis sechs Dentinmassen, eins bis zwei Schmelzmassen, eine transparente Masse und fünf bis zehn stark pigmentierte keramische Malfarben.

6. Farbsystem gemäß Anspruch 5, dadurch gekennzeichnet, daß es eine einzige Opaquermasse, bis zu vier Dentinmassen, eine einzige Schmelzmasse, eine transparente Masse und fünf bis zehn stark pigmentierte keramische Malfarben umfaßt.